# EUROPEAN PATENT APPLICATION

(11) **EP 4 624 455 A1**
(43) Date of publication of application: **01.10.2025**
(21) Application number: 23893889.8
(22) Date of filing: 22.11.2023
(51) Int. Cl.: C07D 209/52

(54) **PREPARATION METHOD FOR CHIRAL PYRROLE DERIVATIVE AND INTERMEDIATE THEREOF**

(30) Priority: 23.11.2022 CN 202211472493
(71) Applicant: Kangbaida (Sichuan) Biotechnology Co., Ltd., Chengdu, Sichuan 610000 (CN)
(72) Inventor: YUAN, Yonghai, Chengdu, Sichuan 610000 (CN); WEI, Yonggang, Chengdu, Sichuan 610000 (CN); JIA, Zhilong, Chengdu, Sichuan 610000 (CN); SUN, Yi, Chengdu, Sichuan 610000 (CN)
(74) Representative: Kador & Partner Part mbB
(86) International application number: PCT/CN2023/133212
(87) International publication number: WO 2024/109801

(57) **Abstract**

The present disclosure relates to a preparation method for a chiral pyrrole derivative and an intermediate thereof.

## Description

### Technical Field

The present disclosure relates to a synthesis method for a chiral pyrrole derivative.

### Background Art

Toll-like receptors (TLRs) belong to a family of molecular pattern recognition receptors widely distributed in different tissues for monitoring and recognizing different pathogen-associated molecular patterns (PAMPs) and damage-associated molecular patterns (DAMPs), and play a crucial role in both innate and adaptive immunity.

TLRs are type I transmembrane proteins. So far, 13 TLR family members have been discovered, 10 of which are present in humans. TLR1, TLR2, TLR4, TLR5, TLR6, TLR10 and TLR11, which reside at the cell membrane, can recognize substances such as lipids and lipoproteins of microorganisms. TLR3, TLR7, TLR8 and TLR9, which are located in intracellular vesicle structures (such as lysosomes, endosomes and endoplasmic reticulum), can recognize nucleic acids of microorganisms.

TLR7 and TLR8 are most similar to each other in terms of sequence and function. A large number of studies have shown that the activation of TLR7/8 can elicit a type I interferon response and a variety of inflammatory responses, and in the case of autoimmune diseases such as systemic lupus erythematosus (SLE), the abnormal sustained activation of TLR7/8 leads to the deterioration of the disease state. Therefore, it is desired to create a new approach for the treatment of autoimmune diseases by developing compounds with selectivity and potent inhibitory activity, which suppresses overactivated immune responses by antagonizing TLR7/8.

### Summary of the Invention

The present disclosure provides a preparation method for a chiral pyrrole derivative that can be used in the synthesis of TLR inhibitors and an intermediate thereof.

One or more embodiments of the present disclosure provide a preparation method for a compound represented by formula (I) or a stereoisomer thereof, wherein: 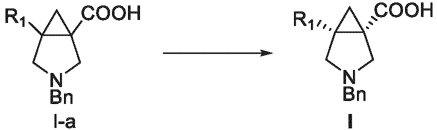 in a reaction solvent, a chiral amine is added to the compound of formula (I-a) to give the compound of formula (I); the reaction solvent is one or more selected from the group consisting of ethyl acetate, methanol, ethanol, isopropanol, acetone, dichloromethane, chloroform, 1,2-dichloroethane, methyl tert-butyl ether, toluene, acetonitrile, tetrahydrofuran and 1,4-dioxane, preferably ethyl acetate; the chiral amine is selected from (1*R*,2*S*)-(-)-2-amino-1,2-diphenylethanol, (*R*)-1-(1-naphthyl)ethylamine, (1*S*,2*R*)-(-)-1-amino-2-indanol, (*R*)-1-(1-naphthyl)ethylamine, (*R*)-1-(2-naphthyl)ethylamine, cinchonidine or quinidine,
wherein:
R₁ is selected from C₁₋₆ alkyl or 3- to 10-membered cycloalkyl, the C₁₋₆ alkyl or 3- to 10-membered cycloalkyl optionally being further substituted with one or more halogens;
preferably, R₁ is CF₃.

One or more embodiments of the present disclosure provide a preparation method for the compound of formula (I-a) or a stereoisomer thereof, wherein: 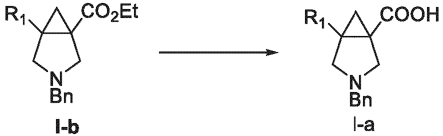 in a reaction solvent, a strong base is added to the compound of formula (I-b) for reaction, and the compound of formula (I-a) is prepared after adjusting the pH with at least one selected from the group consisting of concentrated hydrochloric acid, tartaric acid, citric acid, potassium hydrogen sulfate, potassium dihydrogen phosphate, dipotassium hydrogen phosphate, sodium dihydrogen phosphate, disodium hydrogen phosphate, acetic acid and trifluoroacetic acid, preferably concentrated hydrochloric acid; the reaction solvent is selected from a mixed solvent of THF/H₂O/methanol; and the strong base is one or more selected from the group consisting of lithium hydroxide, sodium hydroxide, potassium hydroxide, potassium trimethylsilanolate and potassium triethylsilanolate, preferably lithium hydroxide (LiOH).

One or more embodiments of the present disclosure provide a preparation method for a pyrrole derivative represented by formula (I) or a stereoisomer thereof, wherein:

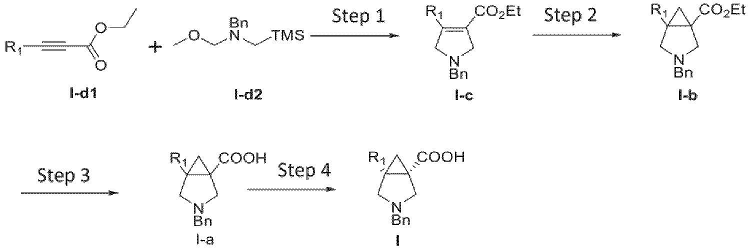

Step 1: in a solution containing one or more solvents selected from the group consisting of dichloromethane, chloroform, 1,2-dichloroethane, tetrahydrofuran and 1,4-dioxane, preferably a dichloromethane (DCM) solution, trifluoroacetic acid, acetic acid or hydrochloric acid (preferably trifluoroacetic acid) is added to the compounds of formulas (I-d1) and (I-d2) for reaction to give the compound of formula (I-c).

Step 2: the compound of formula (I-c) is added to a reaction system of a cyclopropanation reagent, a strong base and a reaction solvent to give the compound of formula (I-b), wherein the cyclopropanation reagent is selected from trimethylsulfoxonium iodide or trimethylsulfonium iodide; the strong base is selected from potassium tert-butanolate or sodium hydride; and the reaction solvent is one or more selected from the group consisting of dimethyl sulfoxide, tetrahydrofuran and 1,4-dioxane, preferably dimethyl sulfoxide (DMSO).

Step 3: in a reaction solvent, a strong base is added to the compound of formula (I-b) for reaction, and the compound of formula (I-a) is prepared after adjusting the pH with one or more selected from the group consisting of concentrated hydrochloric acid, tartaric acid, citric acid, potassium hydrogen sulfate, potassium dihydrogen phosphate, dipotassium hydrogen phosphate, sodium dihydrogen phosphate, disodium hydrogen phosphate, acetic acid and trifluoroacetic acid, preferably concentrated hydrochloric acid, the reaction solvent is selected from mixed solvents of THF/H₂O/methanol; and the strong base is one or more selected from the group consisting of lithium hydroxide, sodium hydroxide, potassium hydroxide, potassium trimethylsilanolate and potassium triethylsilanolate, preferably lithium hydroxide (LiOH).

Step 4: in a reaction solvent, a chiral amine is added to the compound of formula (I-a) to give the compound of formula (I), wherein the reaction solvent is one or more selected from the group consisting of ethyl acetate, methanol, ethanol, isopropanol, acetone, dichloromethane, chloroform, 1,2-dichloroethane, methyl tert-butyl ether, toluene, acetonitrile, tetrahydrofuran and 1,4-dioxane, preferably ethyl acetate; and the chiral amine is selected from (1R,2S)-(-)-2-amino-1,2-diphenylethanol, (R)-1-(1-naphthyl)ethylamine, (1S,2R)-(-)-1-amino-2-indanol, (*R*)-1-(1-naphthyl)ethylamine, (*R*)-1-(2-naphthyl)ethylamine, cinchonidine or quinidine.

In this case,
R₁ is C₁₋₆ alkyl, the C₁₋₆ alkyl optionally being further substituted with one or more halogens;
preferably, R₁ is CF₃.

One or more embodiments of the present disclosure provide a preparation method for a pyrrole derivative represented by formula (I-h) or a stereoisomer thereof, wherein:

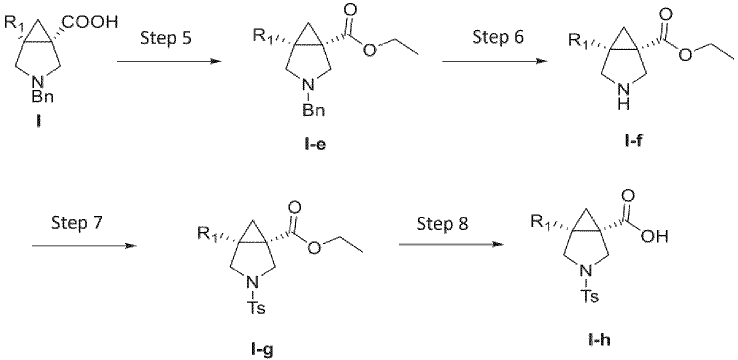

Step 5: in a reaction solvent, an acylchlorination reagent is added to formula (I) to give the compound (I-e), wherein the reaction solvent is one or more selected from the group consisting of dichloromethane, chloroform and 1,2-dichloroethane, preferably dichloromethane (DCM), and the acylchlorination reagent is selected from thionyl chloride or oxalyl chloride.

Step 6: in a reaction solvent, a metal catalyst and a reducing agent are added to formula (I-e) for reaction to give the compound (I-f), wherein the reaction solvent is one or more selected from the group consisting of methanol, ethanol and isopropanol, preferably methanol; the metal catalyst is selected from palladium on carbon and palladium hydroxide; and the reducing agent is selected from hydrogen and ammonium formate.

Step 7: in a reaction solvent and in the presence of an organic base, p-toluenesulfonyl chloride is added to formula (I-f) for reaction to give the compound (I-g), wherein the organic base is one or more selected from the group consisting of triethylamine, diisopropylethylamine, potassium carbonate and cesium carbonate, preferably triethylamine; and the reaction solvent is one or more selected from the group consisting of dichloromethane, chloroform, 1,2-dichloroethane, dimethyl sulfoxide, N,N-dimethylformamide, tetrahydrofuran and 1,4-dioxane, preferably dichloromethane (DCM).

Step 8: in a reaction solvent, a strong base is added to the compound of formula (I-g) for reaction to give the compound of formula (I-h), wherein the reaction solvent is one or more selected from the group consisting of tetrahydrofuran/water, tetrahydrofuran, 1,4-dioxane, water, methanol, ethanol and isopropanol, preferably a mixed solvent of tetrahydrofuran/water (THF/H₂O); and the strong base is one or more selected from the group consisting of lithium hydroxide, sodium hydroxide, potassium hydroxide, potassium trimethylsilanolate and potassium triethylsilanolate, preferably lithium hydroxide (LiOH).

In this case,
R₁ is C₁₋₆ alkyl, the C₁₋₆ alkyl optionally being further substituted with one or more halogens;
preferably, R₁ is CF₃.

One or more embodiments of the present disclosure provide a preparation method for a compound represented by formula (I) or a stereoisomer thereof, wherein: 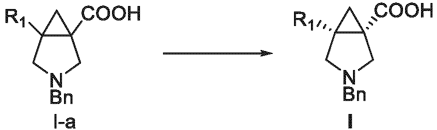
in a reaction solvent, a chiral amine is added to the compound of formula (I-a) to give the compound of formula (I), wherein the reaction solvent is selected from ethyl acetate, and the chiral amine is selected from (1*R*,2*S*)-(-)-2-amino-1,2-diphenylethanol, (*R*)-1-(1-naphthyl)ethylamine, (1*S*,2*R*)-(-)-1-amino-2-indanol, (*R*)-1-(1-naphthyl)ethylamine, (*R*)-1-(2-naphthyl)ethylamine, cinchonidine or quinidine,
In this case,
R₁ is selected from C₁₋₆ alkyl or 3- to 10-membered cycloalkyl, the C₁₋₆ alkyl or 3- to 10-membered cycloalkyl optionally being further substituted with one or more halogens;
preferably, R₁ is CF₃.

One or more embodiments of the present disclosure provide a preparation method for the compound of formula (I-a) or a stereoisomer thereof, wherein: 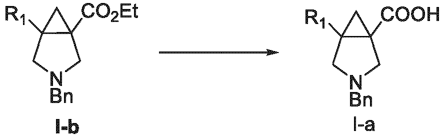 in a reaction solvent, a strong base is added to the compound of formula (I-b) for reaction, and the compound of formula (I-a) is prepared after adjusting the pH with concentrated hydrochloric acid; the reaction solvent is selected from mixed solvents of THF/H₂O/methanol; and the strong base is selected from LiOH.

One or more embodiments of the present disclosure provide a preparation method for a pyrrole derivative represented by formula (I) or a stereoisomer thereof, wherein:

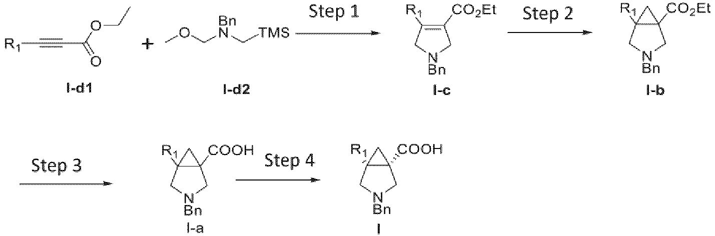

Step 1: in a DCM solution, trifluoroacetic acid is added to the compounds of formulas (I-d1) and (I-d2) for reaction to give the compound of formula (I-c).

Step 2: the compound of formula (I-c) is added to a reaction system of a cyclopropanation reagent, a strong base and a reaction solvent to give the compound of formula (I-b), wherein the cyclopropanation reagent is selected from trimethylsulfoxonium iodide or trimethylsulfonium iodide; the strong base is selected from potassium tert-butanolate or sodium hydride; and the reaction solvent is selected from DMSO.

Step 3: in a reaction solvent, a strong base is added to the compound of formula (I-b) for reaction, and the compound of formula (I-a) is prepared after adjusting the pH with concentrated hydrochloric acid; the reaction solvent is selected from mixed solvents of THF/H₂O/methanol; and the strong base is selected from LiOH.

Step 4: in a reaction solvent, a chiral amine is added to the compound of formula (I-a) to give the compound of formula (I), wherein the reaction solvent is selected from ethyl acetate, and the chiral amine is selected from (1R,2S)-(-)-2-amino-1,2-diphenylethanol, (R)-1-(1-naphthyl)ethylamine, (1S,2R)-(-)-1-amino-2-indanol, (*R*)-1-(1-naphthyl)ethylamine, (*R*)-1-(2-naphthyl)ethylamine, cinchonidine or quinidine.

In this case,
R₁ is C₁₋₆ alkyl, the C₁₋₆ alkyl optionally being further substituted with one or more halogens;
preferably, R₁ is CF₃.

One or more embodiments of the present disclosure provide a preparation method for a pyrrole derivative represented by formula (I-h) or a stereoisomer thereof, wherein:

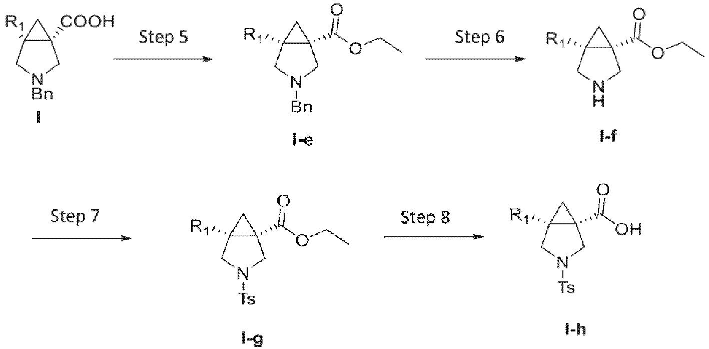

Step 5: in a reaction solvent, an acylchlorination reagent is added to formula (I) to give the compound (I-e), wherein the reaction solvent is selected from DCM; and the acylchlorination reagent is selected from thionyl chloride or oxalyl chloride.

Step 6: in a reaction solvent, a metal catalyst and a reducing agent are added to formula (I-e) for reaction to give the compound (I-f), wherein the reaction solvent is selected from methanol; the metal catalyst is selected from palladium on carbon and palladium hydroxide; and the reducing agent is selected from hydrogen and ammonium formate.

Step 7: in a reaction solvent and in the presence of an organic base, p-toluenesulfonyl chloride is added to formula (I-f) for reaction to give the compound (I-g), wherein the organic base is selected from triethylamine; and wherein the reaction solvent is selected from DCM; and

Step 8: in a reaction solvent, a strong base is added to the compound of formula (I-g) for reaction to give the compound of formula (I-h), wherein the reaction solvent is selected from mixed solvents of THF/H₂O; and the strong base is selected from LiOH.

In this case,
R₁ is C₁₋₆ alkyl, the C₁₋₆ alkyl optionally being further substituted with one or more halogens;
preferably, R₁ is CF₃.

One or more embodiments of the present disclosure provide a preparation method for an intermediate of the compound of formula (I) or formula (I-h), or a stereoisomer thereof,
wherein the intermediate is selected from: 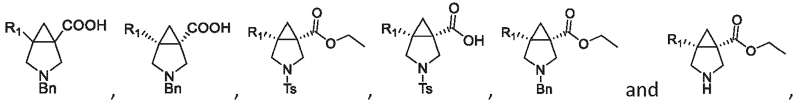
wherein R₁ is as defined above,
with the proviso that the intermediate is not

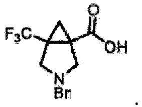

Unless stated to the contrary, the terms used in the description and claims have the following meanings.

The "stereoisomer" refers to an isomer produced as a result of different spatial arrangement of atoms in molecules, including cis-trans isomers, enantiomers and conformational isomers.

"Optional" or "optionally" or "alternative" or "alternatively" means that the events or conditions subsequently described may but not necessarily occur, and the description includes the case where the events or conditions occur and do not occur. For example, "heterocyclyl alternatively substituted with alkyl" means that the alkyl may but not necessarily exist, and the description includes the case where the heterocyclyl is substituted with alkyl and the case where the heterocyclyl is not substituted with alkyl.

A base refers to a compound that ionizes in an aqueous solution to give OH-, or a compound that is able to accept a proton. Examples of the strong base of the present disclosure include cesium carbonate, sodium hydroxide, potassium hydroxide, barium hydroxide, cesium hydroxide, lithium hydroxide, etc.

An organic base of the present disclosure refers to a compound containing a nitrogen atom, such as an amine compound and a nitrogen-containing heterocyclic compound. Nonlimiting examples include: sodium methanolate, potassium ethanolate, potassium tert-butanolate, butyllithium, phenyllithium, Grignard reagents, quaternary ammonium hydroxides, pyridine, and lithium amide compounds (e.g., lithium diisopropylamide (LDA), and lithium hexamethyldisilazide (LiHMDS)). Examples of the organic base chosen in the present disclosure include sodium tert-butanolate, potassium tert-butanolate, triethylamine, N,N-diisopropylethylamine, 1,8-diazabicyclo[5.4.0]undec-7-ene, 4-dimethylaminopyridine, sodium tert-amylate, etc.

### Brief Description of the Drawings

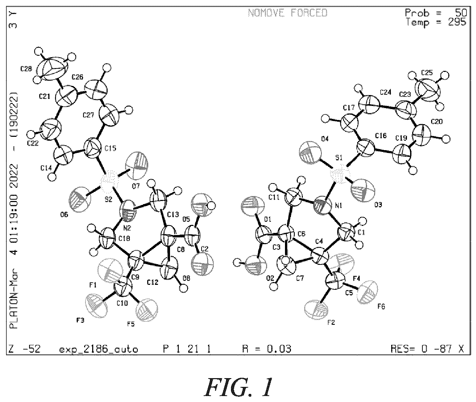

FIG. 1 shows the X-ray single crystal diffractogram of compound 2.

### Detailed Description of Embodiments

The technical solutions of the present disclosure will be described in detail by the following examples, but the scope of protection of the present disclosure includes but is not limited thereto.
DMSO: dimethyl sulfoxide;
DCM: dichloromethane;
THF: tetrahydrofuran.

### Examples

### Example 1

### (1S,5R)-3-benzyl-5-(trifluoromethyl)-3-azabicyclo[3.1.0]hexane-1-carboxylic acid Compound 1

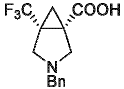

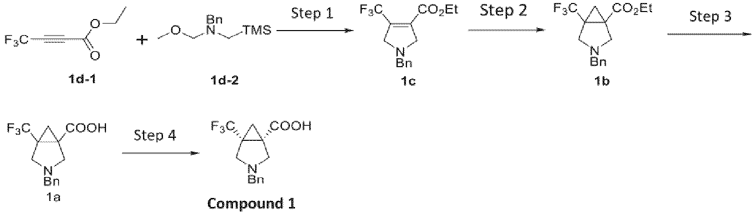

### Step 1:

### Ethyl 1-benzyl-4-(trifluoromethyl)-2,5-dihydro-1H-pyrrole-3-carboxylate 1c

The compound ethyl 4,4,4-trifluoro-2-butynoate **1d-1** (200 g, 1.2 mol) was added to a clean three-necked flask and mixed homogeneously with dichloromethane (2 L), and then trifluoroacetic acid (1.36 g, 12 mmol) was added. The compound N-(methoxymethyl)-N-(trimethylsilylmethyl)benzylamine **1d-2** (284 g, 1.2 mol) was mixed homogeneously with dichloromethane (400 ml), the resulting mixture was added dropwise to the above solution at room temperature (within about 2 h), and after the addition was completed, the reaction was carried out for 1 h further at room temperature. Upon the completion of the reaction under monitoring by LC-MS, water (2 L) was added to the reaction system, stirred and mixed homogeneously, and the resulting mixture was left to stand for layering. The liquid was separated by using a separating funnel. The organic phase was collected, and the aqueous phase was then extracted with dichloromethane (1 L). The organic phases were combined, dried and concentrated at reduced pressure to give the compound ethyl 1-benzyl-4-(trifluoromethyl)-2,5-dihydro-1H-pyrrole-3-carboxylate **3** (crude product, 361 g, yellow liquid).

**¹H NMR** (400 MHz, DMSO-*d*₆) δ 7.34-7.24 (m, 5H), 4.19 (q, 2H), 3.79-3.78 (m, 6H), 1.20 (t, 3H).

**LC-MS** m/z (ESI) = 300.1 [M+1].

### Step 2:

### Ethyl-3-benzyl-5-(trifluoromethyl)-3-azabicyclo[3.1.0]hexane-1-carboxylate 1b

The compound trimethylsulfoxonium iodide (264 g, 1.2 mol) was added to a clean three-necked flask and mixed with DMSO (1.5 L) homogeneously, purged with nitrogen three times. Potassium tert-butanolate (142 g, 1.26 mol) was added at room temperature, and stirred for further 30 min. The compound ethyl 1-benzyl-4-(trifluoromethyl)-2,5-dihydro-1H-pyrrole-3-carboxylate **1c** (361 g, 1.2 mol) was mixed with DMSO (500 ml) homogeneously, the resulting mixture was added dropwise to the above solution (within about 1 h), and after the addition is completed, the reaction was carried out for 1 h further. Upon the completion of the reaction under monitoring by LC-MS, a saturated solution of ammonium chloride (500 ml) was added to the reaction system and stirred for 10 min. Water (500 ml) and ethyl acetate (2 L) were then added and mixed homogeneously under stirring. The resulting mixture was left to stand for layering. The liquid was separated by using a separating funnel. The aqueous phase was removed, and the organic phase was washed with water twice (1 L * 2). The aqueous phases were combined and then stripped once with ethyl acetate (1 L). The ethyl acetate phases were combined, dried with anhydrous sodium sulfate and concentrated at reduced pressure to give the compound ethyl 3-benzyl-5-(trifluoromethyl)-3-azabicyclo[3.1.0]hexane-1-carboxylate **1b** (crude product, 347 g, brown liquid).

**¹H NMR** (400 MHz, DMSO-*d*₆) δ 7.34-7.24 (m, 5 H), 4.11 (q, 2H), 3.66 (s, 2H), 3.08-3.01 (m, 2H), 2.86-2.82 (m, 1H), 2.65-2.62 (m, 1H), 1.82-1.79 (m, 2H), 1.15 (t, 3H).

**LC-MS** m/z (ESI) = 314.1 [M+1].

### Step 3:

### 3-Benzyl-5-(trifluoromethyl)-3-azabicyclo[3.1.0]hexane-1-carboxylic acid 1a

The compound ethyl-3-benzyl-5-(trifluoromethyl)-3-azabicyclo[3.1.0]hexane-1-carboxylate **1b** (347 g, 1.1 mol) was mixed with tetrahydrofuran (600 ml), water (600 ml) and methanol (60 ml) homogeneously, then lithium hydroxide monohydrate (185 g, 4.4 mol) was added, and the reaction was carried out at 60°C for 1 h. Upon the completion of the reaction under monitoring by LC-MS, the mixture was cooled to room temperature, and the organic solvent was removed at reduced pressure. The aqueous phase was extracted with dichloromethane (1 L), and the organic phase was stripped once with water (500 ml). The aqueous phases were combined, and the pH was adjusted to 3-4 with concentrated hydrochloric acid. The aqueous phase was then extracted with ethyl acetate (1 L * 6 times). The organic phases were combined, dried with anhydrous sodium sulfate, and then concentrated at reduced pressure to give the compound 3-benzyl-5-(trifluoromethyl)-3-azabicyclo[3.1.0]hexane-1-carboxylic acid **1a** (crude product, 245 g, brown liquid).

LC-MS m/z (ESI) = **286.10** [M+1].

### Step 4:

### (1S,5R)-3-benzyl-5-(trifluoromethyl)-3-azabicyclo[3.1.0]hexane-1-carboxylic acid Compound 1

3-Benzyl-5-(trifluoromethyl)-3-azabicyclo[3.1.0]hexane-1-carboxylic acid **1a** (24.0 g, 0.084 mol) was added to a reaction flask and dissolved in ethyl acetate (500 mL). (1R,2S)-(-)-2-amino-1,2-diphenylethanol (18.0 g, 0.084 mol) was added at room temperature, stirred for 10 min, so that a large amount of a white solid precipitated. The mixture was heated to reflux, and ethyl acetate (600 mL) was replenished until the solution was completely clear. The solution was cooled to room temperature, so that a solid precipitated, which was then filtrated to obtain 19 g of a white solid. The resulting solid was subsequently dissolved in ethyl acetate, and 2 N hydrochloric acid was added to adjust the pH to 3-4. The organic phase was separated, dried with anhydrous sodium sulfate, and then concentrated at reduced pressure to give (15,5R)-3-benzyl-5-(trifluoromethyl)-3-azacyclo[3.1.0]hexane-1-carboxylic acid **Compound 1** (10.8 g, white solid, yield: 45%, 96% ee). Analytical method: The value of ee was obtained by means of chiral HPLC analysis, with a chiral column of AD, a mobile phase of V(n-hexane)/V(methanol) = 95/5, a flow rate of 1 mL/min, and retention time of tₘᵢₙₒᵣ = 2.032 min and tₘₐⱼₒᵣ = 2.607 min.

LC-MS m/z (ESI) = **286.10** [M+1].

The resolution results of other chiral amines are shown in Table 1.

**Table 1 Resolution results of other chiral amines**

| Name of chiral amine | Structure | Yield (%) | ee (%) |
|---|---|---|---|
| (R)-1-(1-naphthyl)ethylamine | 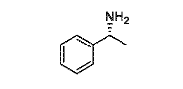 | 38 | 70 |
| (1S,2R)-(-)-1-amino-2-indanol | 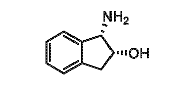 | 0 | - |
| (*R*)-1-(1-naphthyl)ethylamine | 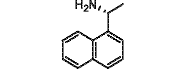 | 30 | -77 |
| (*R*)-1-(2-naphthyl)ethylamine | 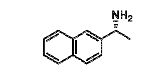 | 63 | -37 |
| Cinchonidine | 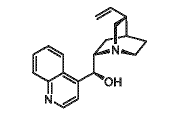 | 32 | 75 |
| Quinidine | 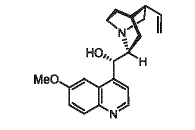 | 0 | - |

### Example 2

### (1S,5R)-3-tosyl-5-(trifluoromethyl)-3-azabicyclo[3.1.0]hexane-1-carboxylic acid Compound 2

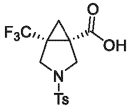

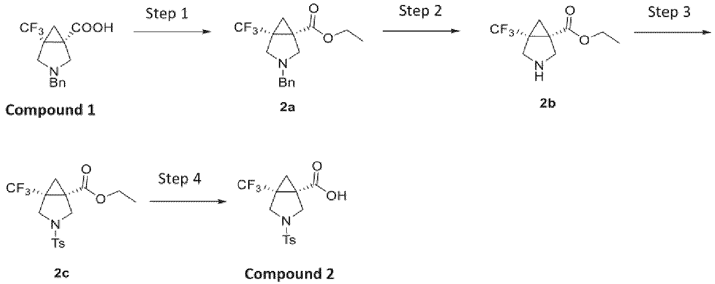

### Step 1:

### (1S,5R)-ethyl-3-benzyl-5-(trifluoromethyl)-3-azabicyclo[3.1.0]hexane-1-carboxylate 2a

(1*S*,5*R*)-3-benzyl-5-(trifluoromethyl)-3-azacyclo[3.1.0]hexane-1-carboxylic acid **Compound 1** (350 mg, 1.23 mmol) was dissolved in dichloromethane (20 mL). Sulfoxide chloride (5 mL) was then added and stirred under reflux by heating for 30 min. Subsequently, the solvent was concentrated, and ethanol (10 mL) was added. The resulting reaction solution was extracted with ethyl acetate, washed with saturated brine (15 mL) and dried with anhydrous sodium sulfate. The organic phase was subjected to rotary evaporation, followed by purification by means of silica gel column chromatography, thereby obtaining the target compound (15,5R)-ethyl-3-benzyl-5-(trifluoromethyl)-3-azabicyclo[3.1.0]hexane-1-carboxylate **2a** (colorless oily liquid, 300 mg, yield: 78%).

**LC-MS** m/z (ESI) = **314.13** [M+1].

### Step 2:

### Ethyl-(1S,5R)-5-(trifluoromethyl)-3-azabicyclo[3.1.0]hexane-1-carboxylate 2b

(1*S*,5*R*)-ethyl-3-benzyl-5-(trifluoromethyl)-3-azabicyclo[3.1.0]hexane-1-carboxylate **2a** (300 mg, 0.96 mmol) was dissolved in 5 mL of methanol, palladium on carbon (30 mg, 0.19 mmol) and ammonium formate (241.5 mg, 3.83 mmol) were then added successively and heated under reflux for 6 h. Upon the completion of the reaction under monitoring by TLC, the resulting reaction solution was concentrated to give the target compound ethyl-(1S,5R)-5-(trifluoromethyl)-3-azabicyclo[3.1.0]hexane-1-carboxylate **2b,** which was used directly in the next step.

**LC-MS** m/z (ESI) = **224.08** [M+1].

### Step 3:

### Ethyl-(1S,5R)-3-tosyl-5-(trifluoromethyl)-3-azabicyclo[3.1.0]hexane-1-carboxylate 2c 9

Ethyl-(15,5R)-5-(trifluoromethyl)-3-azabicyclo[3.1.0]hexane-1-carboxylate **2b** was dissolved in dichloromethane (20 mL), then triethylamine (0.4 mL, 2.88 mmol) and p-toluenesulfonyl chloride (273.6 mg, 1.44 mmol) were added and stirred at room temperature for 2 h. Upon the completion of the reaction under monitoring by TLC, the resulting reaction solution was concentrated and then purified by means of silica gel column chromatography to give ethyl-(1S,5R)-3-tosyl-5-(trifluoromethyl)-3-azabicyclo[3.1.0]hexane-1-carboxylate 2c (colorless oil, 258 mg, two-step yield: 72%)

**LC-MS** m/z (ESI) = **378.09** [M+1].

### Step 4:

### (1S,5R)-3-tosyl-5-(trifluoromethyl)-3-azabicyclo[3.1.0]hexane-1-carboxylic acid Compound 2

Ethyl-(1*S*,5*R*)-3-tosyl-5-(trifluoromethyl)-3-azabicyclo[3.1.0]hexane-1-carboxylate **2c** (258 mg, 0.69 mmol) was dissolved in a tetrahydrofuran solution (10 mL). An aqueous solution (10 mL) of anhydrous lithium hydroxide (165 mg, 6.9 mmol) was dripped into the reaction solution and stirred at room temperature overnight. After the completion of the reaction, tetrahydrofuran was removed by means of rotary evaporation, followed by extraction with ethyl acetate, and the aqueous phase was retained. The aqueous phase was adjusted to the pH of 3-4 with a 2 M aqueous hydrochloric acid solution, extracted with ethyl acetate, washed with saturated brine and then dried with anhydrous sodium sulfate. The solvent was removed in vacuum. Purification by means of silica gel column chromatography resulted in (1*S*,5*R*)-3-tosyl-5-(trifluoromethyl)-3-azabicyclo[3.1.0]hexane-1-carboxylic acid **Compound 2** (white solid, 158 mg, yield: 66%).

**LC-MS** m/z (ESI) = **350.06** [M+1].

**¹H NMR** (400 MHz, DMSO) *δ* = 13.34 (s, 1H), 7.74 (d, 2H), 7.50 (d, 2H), 3.73 (d, 1H), 3.66 (d, 1H), 3.39 (d, 1H), 3.24 (d, 1H), 2.43 (s, 3H), 1.93 (d, 1H), 1.32 (d, 1H).

### Determination of the absolute configuration of compound 2

Compound 2 (100 mg) was dissolved in 6 mL solvent (petroleum ether : ethyl acetate = 5 : 1), followed by spontaneous volatilization under room temperature until single crystals grew. The absolute configuration thereof was measured using an Xtalab Synergy single-crystal diffractometer with a rotating anode from Rigaku Corporation, Japan. The single crystal data are shown in Table 2.

**Table 2 Single crystal data of Compound 2**

| | |
|---|---|
| Empirical formula | C₁₄H₁₄F₃NO₄S |
| Formula weight | 349.32 |
| Temperature/K | 294.15 |
| Crystal system | Monoclinic |
| Space group | P2₁ |
| a/Å | 12.48010(10) |
| b/Å | 5.78530(10) |
| c/Å | 22.0239(2) |
| α/° | 90 |
| β/° | 103.3450(10) |
| γ/° | 90 |
| Volume/Å³ | 1547.21(3) |
| Z | 2 |
| ρ_{calc}g/cm³ | 1.500 |
| µ//mm⁻¹ | 2.358 |
| F(000) | 720.0 |
| Crystal size/mm³ | 0.2 × 0.16 x 0.15 |
| Radiation | CuKα (λ = 1.54184) |
| 2θ range for data collection/° | 4.124 to 153.926 |
| Index ranges | -15 ≤ h ≤ 15, -7 ≤ k ≤ 5, -27 ≤ I ≤ 27 |
| Reflections collected | 15832 |
| Independent reflections | **5521** [Rᵢₙₜ = 0.0255, R_{sigma} = 0.0238] |
| Data/restraints/parameters | 5521/1/425 |
| Goodness-of-fit on F² | 1.038 |
| Final R indexes [I >= 2σ (I)] | R₁ = 0.0341, wR₂ = 0.0922 |
| Final R indexes [all data] | R₁ = 0.0350, wR₂ = 0.0931 |
| Largest diff. Peak/hole / eÅ⁻³ on difference Fourier map | 0.16/-0.31 |
| Flack parameter | -0.006(14) |

Specific embodiments have been described in detail in the description of the present disclosure. A person skilled in the art should recognize that the embodiments described above are exemplary and cannot be construed as limiting the present disclosure. Additionally, a person skilled in the art can make several improvements and modifications to the present disclosure without departing from the principle of the present disclosure, and the technical solutions obtained on the basis of these improvements and modifications also fall within the scope of protection of the claims of the present disclosure.

## Claims

1. A preparation method for a compound represented by formula (I) or a stereoisomer thereof, wherein: 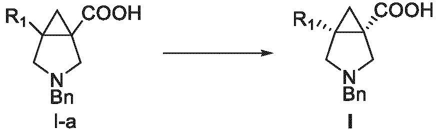
in a reaction solvent, a chiral amine is added to the compound of formula (I-a) to give the compound of formula (I), the reaction solvent is one or more selected from the group consisting of ethyl acetate, methanol, ethanol, isopropanol, acetone, dichloromethane, chloroform, 1,2-dichloroethane, methyl tert-butyl ether, toluene, acetonitrile, tetrahydrofuran and 1,4-dioxane, preferably ethyl acetate; the chiral amine is selected from (1*R*,2*S*)-(-)-2-amino-1,2-diphenylethanol, (*R*)-1-(1-naphthyl)ethylamine, (1*S*,2*R*)-(-)-1-amino-2-indanol, (*R*)-1-(1-naphthyl)ethylamine, (*R*)-1-(2-naphthyl)ethylamine, cinchonidine or quinidine,
wherein
R₁ is selected from C₁₋₆ alkyl or 3- to 10-membered cycloalkyl, the C₁₋₆ alkyl or 3- to 10-membered cycloalkyl optionally being further substituted with one or more halogens; preferably, R₁ is CF₃.

2. A preparation method for a compound represented by formula (I) or a stereoisomer thereof, wherein: 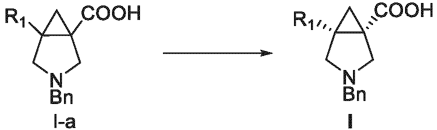
in a reaction solvent, a chiral amine is added to the compound of formula (I-a) to give the compound of formula (I), wherein the reaction solvent is selected from ethyl acetate, and the chiral amine is selected from (1*R*,2*S*)-(-)-2-amino-1,2-diphenylethanol, (*R*)-1-(1-naphthyl)ethylamine, (1*S*,2*R*)-(-)-1-amino-2-indanol, (*R*)-1-(1-naphthyl)ethylamine, (*R*)-1-(2-naphthyl)ethylamine, cinchonidine or quinidine,
wherein:
R₁ is C₁₋₆ alkyl, the C₁₋₆ alkyl optionally being further substituted with one or more halogens;
preferably, R₁ is CF₃.

3. The preparation method for the compound of formula (I-a) or a stereoisomer thereof according to claim 1, wherein: 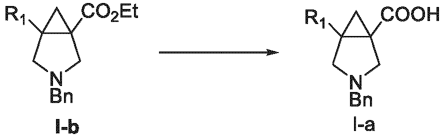 in a reaction solvent, a strong base is added to the compound of formula (I-b) for reaction, and the compound of formula (I-a) is prepared after adjusting the pH with one or more selected from the group consisting of concentrated hydrochloric acid, tartaric acid, citric acid, potassium hydrogen sulfate, potassium dihydrogen phosphate, dipotassium hydrogen phosphate, sodium dihydrogen phosphate, disodium hydrogen phosphate, acetic acid and trifluoroacetic acid, preferably concentrated hydrochloric acid, the reaction solvent is selected from mixed solvents of THF/H₂O/methanol; and the strong base is one or more selected from the group consisting of lithium hydroxide, sodium hydroxide, potassium hydroxide, potassium trimethylsilanolate and potassium triethylsilanolate, preferably lithium hydroxide (LiOH).

4. The preparation method for the compound of formula (I-a) or a stereoisomer thereof according to claim 2, wherein: 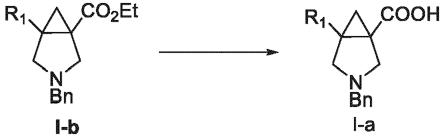 in a reaction solvent, a strong base is added to the compound of formula (I-b) for reaction, and the compound of formula (I-a) is prepared after adjusting the pH with concentrated hydrochloric acid; the reaction solvent is selected from mixed solvents of THF/H₂O/methanol; and the strong base is selected from LiOH.

5. A preparation method for a pyrrole derivative represented by formula (I) or a stereoisomer thereof, comprising 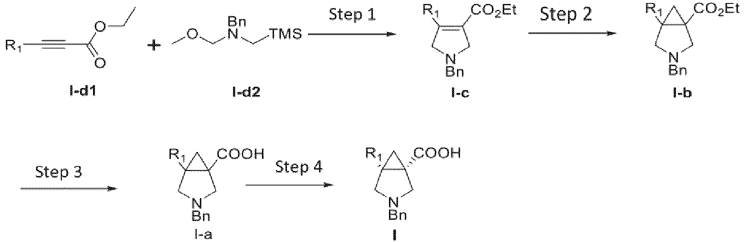
Step 1: in a solution comprising one or more solvents selected from the group consisting of dichloromethane, chloroform, 1,2-dichloroethane, tetrahydrofuran and 1,4-dioxane, preferably a dichloromethane (DCM) solution, trifluoroacetic acid, acetic acid or hydrochloric acid, preferably trifluoroacetic acid, is added to the compounds of formulas (I-d1) and (I-d2) for reaction to give the compound of formula (I-c);
Step 2: the compound of formula (I-c) is added to a reaction system of a cyclopropanation reagent, a strong base and a reaction solvent to give the compound of formula (I-b), wherein the cyclopropanation reagent is selected from trimethylsulfoxonium iodide or trimethylsulfonium iodide; the strong base is selected from potassium tert-butanolate or sodium hydride; and the reaction solvent is one or more selected from the group consisting of dimethyl sulfoxide, tetrahydrofuran and 1,4-dioxane, preferably dimethyl sulfoxide (DMSO);
Step 3: in a reaction solvent, a strong base is added to the compound of formula (I-b) for reaction, and the compound of formula (I-a) is prepared after adjusting the pH with one or more selected from the group consisting of concentrated hydrochloric acid, tartaric acid, citric acid, potassium hydrogen sulfate, potassium dihydrogen phosphate, dipotassium hydrogen phosphate, sodium dihydrogen phosphate, disodium hydrogen phosphate, acetic acid and trifluoroacetic acid, preferably concentrated hydrochloric acid, the reaction solvent is selected from mixed solvents of THF/H₂O/methanol; and the strong base is one or more selected from the group consisting of lithium hydroxide, sodium hydroxide, potassium hydroxide, potassium trimethylsilanolate and potassium triethylsilanolate, preferably lithium hydroxide (LiOH);
Step 4: in a reaction solvent, a chiral amine is added to the compound of formula (I-a) to give the compound of formula (I), wherein the reaction solvent is one or more selected from the group consisting of ethyl acetate, methanol, ethanol, isopropanol, acetone, dichloromethane, chloroform, 1,2-dichloroethane, methyl tert-butyl ether, toluene, acetonitrile, tetrahydrofuran and 1,4-dioxane, preferably ethyl acetate; and the chiral amine is selected from (1R,2S)-(-)-2-amino-1,2-diphenylethanol, (R)-1-(1-naphthyl)ethylamine, (1S,2R)-(-)-1-amino-2-indanol, (*R*)-1-(1-naphthyl)ethylamine, (*R*)-1-(2-naphthyl)ethylamine, cinchonidine or quinidine,
wherein:
R₁ is C₁₋₆ alkyl, the C₁₋₆ alkyl optionally being further substituted with one or more halogens;
preferably, R₁ is CF₃.

6. A preparation method for a pyrrole derivative represented by formula (I) or a stereoisomer thereof, comprising 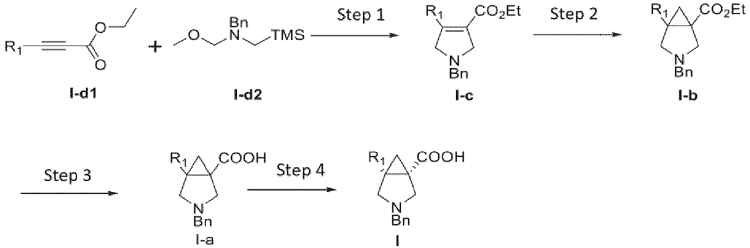
Step 1: in a DCM solution, trifluoroacetic acid is added to the compounds of formulas (I-d1) and (I-d2) for reaction to give the compound of formula (I-c);
Step 2: the compound of formula (I-c) is added to a reaction system of a cyclopropanation reagent, a strong base and a reaction solvent to give the compound of formula (I-b), wherein the cyclopropanation reagent is selected from trimethylsulfoxonium iodide or trimethylsulfonium iodide; the strong base is selected from potassium tert-butanolate or sodium hydride; and the reaction solvent is selected from DMSO;
Step 3: in a reaction solvent, a strong base is added to the compound of formula (I-b) for reaction, and the compound of formula (I-a) is prepared after adjusting the pH with concentrated hydrochloric acid; the reaction solvent is selected from mixed solvents of THF/H₂O/methanol; and the strong base is selected from LiOH;
Step 4: in a reaction solvent, a chiral amine is added to the compound of formula (I-a) to give the compound of formula (I), wherein the reaction solvent is selected from ethyl acetate, and the chiral amine is selected from (1R,2S)-(-)-2-amino-1,2-diphenylethanol, (R)-1-(1-naphthyl)ethylamine, (1S,2R)-(-)-1-amino-2-indanol, (*R*)-1-(1-naphthyl)ethylamine, (*R*)-1-(2-naphthyl)ethylamine, cinchonidine or quinidine,
wherein:
R₁ is C₁₋₆ alkyl, the C₁₋₆ alkyl optionally being further substituted with one or more halogens;
preferably, R₁ is CF₃.

7. A preparation method for a pyrrole derivative represented by formula (I-h) or a stereoisomer thereof, comprising 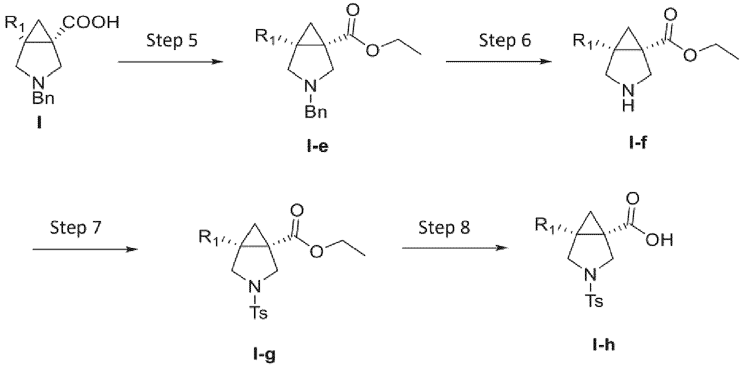
Step 5: in a reaction solvent, an acylchlorination reagent is added to formula (I) to give the compound (I-e), wherein the reaction solvent is one or more selected from the group consisting of dichloromethane, chloroform and 1,2-dichloroethane, preferably dichloromethane (DCM), and the acylchlorination reagent is selected from thionyl chloride or oxalyl chloride;
Step 6: in a reaction solvent, a metal catalyst and a reducing agent are added to formula (I-e) for reaction to give the compound (I-f), wherein the reaction solvent is one or more selected from the group consisting of methanol, ethanol and isopropanol, preferably methanol; the metal catalyst is selected from palladium on carbon and palladium hydroxide; and the reducing agent is selected from hydrogen and ammonium formate;
Step 7: in a reaction solvent and in the presence of an organic base, p-toluenesulfonyl chloride is added to formula (I-f) for reaction to give the compound (I-g), wherein the organic base is one or more selected from the group consisting of triethylamine, diisopropylethylamine, potassium carbonate and cesium carbonate, preferably triethylamine; and the reaction solvent is one or more selected from the group consisting of dichloromethane, chloroform, 1,2-dichloroethane, dimethyl sulfoxide, N,N-dimethylformamide, tetrahydrofuran and 1,4-dioxane, preferably dichloromethane (DCM);
Step 8: in a reaction solvent, a strong base is added to the compound of formula (I-g) for reaction to give the compound of formula (I-h), wherein the reaction solvent is one or more selected from the group consisting of tetrahydrofuran/water, tetrahydrofuran, 1,4-dioxane, water, methanol, ethanol and isopropanol, preferably a mixed solvent of tetrahydrofuran/water (THF/H₂O); and the strong base is one or more selected from the group consisting of lithium hydroxide, sodium hydroxide, potassium hydroxide, potassium trimethylsilanolate and potassium triethylsilanolate, preferably lithium hydroxide (LiOH),
wherein:
R₁ is C₁₋₆ alkyl, the C₁₋₆ alkyl optionally being further substituted with one or more halogens;
preferably, R₁ is CF₃.

8. A preparation method for a pyrrole derivative represented by formula (I-h) or a stereoisomer thereof, comprising 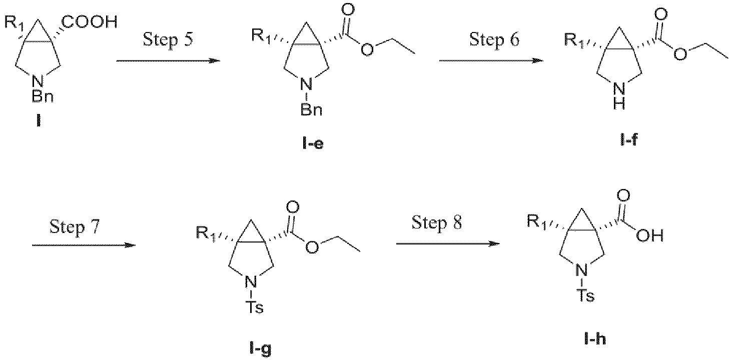
Step 5: in a reaction solvent, an acylchlorination reagent is added to formula (I) to give the compound (I-e), wherein the reaction solvent is selected from DCM; and the acylchlorination reagent is selected from thionyl chloride or oxalyl chloride;
Step 6: in a reaction solvent, a metal catalyst and a reducing agent are added to formula (I-e) for reaction to give the compound (I-f), wherein the reaction solvent is selected from methanol; the metal catalyst is selected from palladium on carbon and palladium hydroxide; and the reducing agent is selected from hydrogen and ammonium formate;
Step 7: in a reaction solvent and in the presence of an organic base, p-toluenesulfonyl chloride is added to formula (I-f) for reaction to give the compound (I-g), wherein the organic base is selected from triethylamine; and wherein the reaction solvent is selected from DCM; and
Step 8: in a reaction solvent, a strong base is added to the compound of formula (I-g) for reaction to give the compound of formula (I-h), wherein the reaction solvent is selected from mixed solvents of THF/H₂O; and the strong base is selected from LiOH,
wherein:
R₁ is C₁₋₆ alkyl, the C₁₋₆ alkyl optionally being further substituted with one or more halogens;
preferably, R₁ is CF₃.

9. An intermediate for the preparation of the compound of formula (I) or formula (I-h) according to any one of claims 1 to 8, or a stereoisomer thereof, wherein the intermediate is selected from: 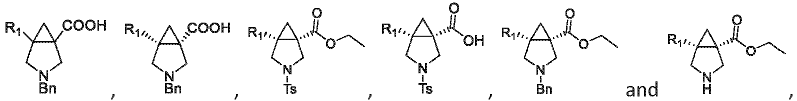 wherein R₁ is as defined in any one of claims 1-8,
with the proviso that the intermediate is not
